# FASCICULE DE BREVET EUROPEEN

(11) **EP 4 362 849 B1**
(45) Date de publication et mention de la délivrance du brevet: **24.12.2025**
(21) Numéro de dépôt: 22740829.1
(22) Date de dépôt: 29.06.2022
(51) Int. Cl.: A61F 2/02, A61L 27/16, A61L 27/20, A61L 27/38, A61L 27/56, C12N 5/071

(54) **MATRICE DE RÉCEPTION DE CELLULES PANCRÉATIQUES ET DISPOSITIF AMÉLIORÉ DE PANCRÉAS ARTIFICIEL**
MATRIX ZUR AUFNAHME PANKREATISCHER ZELLEN UND EINE VERBESSERTE VORRICHTUNG FÜR EIN KÜNSTLICHES PANKREAS
MATRIX FOR THE RECEPTION OF PANCREATIC CELLS AND AN IMPROVED ARTIFICIAL PANCREAS DEVICE

(30) Priorité: 30.06.2021 FR 2107086
(43) Date de publication de la demande: 08.05.2024
(73) Titulaire: Université Grenoble Alpes, 38400 Saint-Martin-d'Hères (FR); Centre Hospitalier Universitaire Grenoble Alpes, 38700 La Tronche (FR); Institut Polytechnique de Grenoble, 38000 Grenoble (FR); Centre National de la Recherche Scientifique, 75016 Paris (FR)
(72) Inventeur: ZEBDA, Abdelkader, 38100 GRENOBLE (FR); CINQUIN, Philippe, 38330 SAINT NAZAIRE LES EYMES (FR); BEN TAHAR, Awatef, 38000 GRENOBLE (FR); TUBBS, Emilie, 38410 VAULNAVEYS-LE-HAUT (FR); BENHAMOU, Pierre-Yves, 38240 MEYLAN (FR); LABLANCHE, Sandrine, 38190 BERNIN (FR)
(74) Mandataire: Hautier IP
(86) Numéro de dépôt international: PCT/EP2022/067873
(87) Numéro de publication internationale: WO 2023/275134

(56) Documents cités:
- EP-A1- 2 964 147
- SE-B- 388 357
- US-A- 5 425 764
- US-A- 5 704 910
- US-A1- 2018 263 238

## Description

### DOMAINE TECHNIQUE DE L'INVENTION

La présente invention concerne le domaine des dispositifs implantables de pancréas artificiel. Elle trouve pour application particulièrement avantageuse la production d'insuline pour le traitement du diabète, et notamment le diabète de type 1.

### ETAT DE LA TECHNIQUE

Le diabète est une maladie à spectre mondial, qui affecte enfants comme adultes et qui nécessite un lourd suivi thérapeutique à vie. En 2016, 8% de la population française était concernée par cette maladie. À l'échelle mondiale, on estime à 422 millions le nombre de personnes diabétiques.

Les diabétiques doivent constamment surveiller leur taux de glucose dans le sang (communément abrégé TGS, pouvant aussi être désigné glycémie) afin de recevoir un traitement en cas d'hypoglycémie (TGS anormalement bas) ou d'hyperglycémie (TGS anormalement haut).

De nombreuses complications peuvent être évitées avec une anticipation de ces taux critiques de glucose. En effet, le diabète peut provoquer des troubles du cœur, des reins, de la rétine ou encore du système neuronal en cas de forte augmentation ou diminution de la glycémie. En outre, il est à noter que les coûts des traitements de ces complications représentent la majorité des dépenses liées à la thérapie du diabète.

Il est donc primordial, d'un point de vue sanitaire et économique, de trouver un moyen de maintenir la glycémie à un taux normal chez les individus diabétiques. Actuellement, il existe des méthodes de contrôle de la glycémie par le patient. Parmi elles, le TGS peut être mesuré de manière ponctuelle par un dispositif électrochimique utilisant des électrodes enzymatiques imprimées sur bandelettes, quantifiant la glycémie à partir d'une goutte de sang. Ces dispositifs, communément appelé point of care devices, permettent aux diabétiques de suivre de manière autonome leur glycémie. Le patient est toutefois amené à se piquer plusieurs fois par jour pour contrôler sa glycémie et ainsi éviter toutes complications du diabète. Ce suivi est contraignant pour le patient et entraîne souvent des pertes de sensibilité dans les zones du corps fréquemment piquées.

En alternative, la transplantation d'îlots pancréatiques est un traitement proposé du diabète de type 1, en particulier pour les patients diabétiques pour lesquels le contrôle et le suivi de la glycémie sont difficiles à mettre en œuvre et provoquent des complications.

Pour réaliser cette transplantation d'îlots pancréatiques, également appelée allo-transplantation d'îlots, les médecins prélèvent généralement des îlots comprenant des cellules pancréatiques bêta saines provenant du pancréas d'un donneur d'organe décédé. Les médecins injectent ensuite les cellules d'îlots saines au patient par le biais d'une veine transportant le sang vers le foie. Une fois fixés sur le foie du patient, ces îlots commencent à produire et à libérer de l'insuline dans le corps du patient. Plusieurs injections de cellules d'îlots greffées sont souvent nécessaires pour arrêter l'utilisation de l'insuline.

Cependant, après explantation, la vascularisation autour des îlots transplantés nécessite un certain temps pendant lequel beaucoup d'îlots se dégradent par manque d'oxygène. On estime la mortalité des îlots d'environ 30 % à 40 % du nombre d'îlots transplantés. En outre, une immunosuppression systémique doit être réalisée chez le patient pour éviter un rejet des îlots transplantés.

Plusieurs approches ont été proposées pour limiter les problèmes liés au manque d'oxygène. Certaines approches comprennent des traitements moléculaires et pharmacologiques, pour protéger les îlots pancréatiques contre le stress hypoxique et les réactions immunitaires. Ces traitements restent toutefois lourds pour le patient et d'une efficacité limitée.

D'autres solutions consistent à encapsuler les îlots dans des matrices polymères les isolant du milieu intérieur du corps. Les îlots sont ainsi protégés des attaques du système immunitaire. Il existe d'une part des approches de microencapsulation des îlots. Du fait de leur taille, les microcapsules restent toutefois difficiles à récupérer pour renouveler les îlots et ainsi pour assurer une délivrance d'insuline à long terme.

Il existe d'autre part des approches de macroencapsulation des îlots. Ces approches consistent de façon générale à mélanger les îlots avec un polymère que l'on fait prendre en masse. Du fait de leur taille, la diffusion des nutriments et de l'oxygène dans les macrocapsules est toutefois limitée, notamment au coeur de la macrocapsule. En outre le bioencrassement en surface induit un isolement de la macrocapsule, limitant encore la diffusion des nutriments et de l'oxygène. La mortalité des îlots reste trop importante pour permettre une bonne délivrance d'insuline.

On connait par ailleurs des dispositifs du type de pancréas artificiel. Le document US 5 425 764 A1 décrit un pancréas artificiel implantable comprenant une chambre contenant les îlots de Langerhans munis de canaux d'entrée et sortie pour fournir les îlots, une chambre ouverte de vascularisation, remplie d'une mousse et d'une membrane semi-perméable séparant les chambres. Ce document tente de répondre au problème d'améliorer la durée de vie des ilots en les protégeant, dans une chambre, des agents inflammatoires par exemple. Cependant, la vascularisation à proximité de la chambre, bien qu'elle vise un apport d'oxygène, elle apporte aussi des molécules impliquées dans les réaction inflammatoires et l'apparition de bio encrassement. Le document US 2018/0263238 décrit également un dispositif d'encapsulation de cellules productrices d'insuline comprend des couches formées d'une première membrane et d'une deuxième membrane, soudées entre elles pour former des canaux. Certains canaux reçoivent des îlots et certains canaux sont exempts d'îlots pour former des canaux de transport de fluide permettant d'apporter des nutriments aux îlots. Ce dispositif bien qu'essayant de répondre à la problématique de l'apport de nutriments aux ilots ne permet pas une grande efficacité en ce que les nutriments sont apportés dans des canaux voisins des canaux contenant les ilots, mais diffusent dans le milieu extérieur qui est volontairement ouvert à la vascularisation. Ainsi, l'apport des nutriments vers les ilots passe d'abord par ce milieu qui vient appauvrir la quantité de nutriments pouvant diffuser à nouveau vers les ilots contenus dans d'autres canaux.

Un objet de la présente invention est donc de proposer une solution permettant une délivrance d'insuline améliorée par rapport aux solutions existantes.

Les autres objets, caractéristiques et avantages de la présente invention apparaîtront à l'examen de la description suivante et des dessins d'accompagnement. Il est entendu que d'autres avantages peuvent être incorporés.

### RESUME DE L'INVENTION

Pour atteindre cet objectif, selon un mode de réalisation on prévoit une matrice de réception de cellules pancréatiques comprenant :
- une paroi semi-perméable délimitant au moins en partie un volume intérieur,
- un corps poreux, de préférence à base d'au moins un polymère, disposé dans le volume intérieur, comprenant :
   ∘ un premier jeu de cavité comprenant des cellules pancréatiques,
   ∘ un deuxième jeu de cavité exempt de cellule pancréatique,
   ∘ le premier jeu de cavité et le deuxième jeu de cavité n'étant pas raccordés fluidiquement l'un à l'autre.

La disposition des cellules pancréatiques dans la ou les cavités du corps poreux permet de contrôler la répartition des cellules dans le corps poreux, par opposition aux solutions de macroencapsulation où les cellules sont figées dans une matrice polymère que l'on a fait prendre en masse. Ainsi, la diffusion des nutriments et des gaz jusqu'à chaque cavité comprenant des cellules peut être améliorée. La ou les cavités laissées exemptes de cellules créent en outre des chemins de diffusion des nutriments et des gaz dans la matrice, et forment une ou des zones de réserve de nutriment et de gaz dans la matrice. Par synergie, la matrice limite et de préférence évite la déplétion en nutriment et en gaz des cellules pancréatiques. Leur mortalité est donc diminuée, permettant une meilleure délivrance d'insuline.

Un deuxième aspect de l'invention concerne un dispositif de pancréas artificiel destiné à être implanté dans le corps humain ou animal et comprenant la matrice de réception de cellules pancréatiques selon le premier aspect.

Un troisième aspect de l'invention concerne un procédé de préparation de la matrice de réception de cellules pancréatiques comprenant :
- une fourniture d'une matrice apte à former la matrice de réception selon le premier aspect,
- un ensemencement du premier jeu de cavité de ladite matrice par des cellules pancréatiques.

La matrice apte à former la matrice de réception peut comprendre au moins le corps poreux comprenant :
- un premier jeu de cavité destiné à comprendre des cellules pancréatiques, et plus particulièrement exempt de cellules pancréatiques,
- un deuxième jeu de cavité exempt de cellule pancréatique,
- le premier jeu de cavité et le deuxième jeu de cavité n'étant pas raccordés fluidiquement l'un à l'autre exempt de cellules pancréatiques.

La paroi semi-perméable peut par exemple être ajoutée après l'ensemencement des cellules pancréatiques. La matrice apte à former la matrice de réception peut être la matrice de réception selon le premier aspect, exempte de cellules pancréatiques. Plus particulièrement le premier jeu de cavité peut être exempt de cellules pancréatiques.

Un troisième aspect de l'invention concerne un procédé de délivrance d'insuline comprenant l'implantation du dispositif de pancréas artificiel selon le deuxième aspect, dans un corps humain ou animal.

Selon un autre aspect, l'invention concerne une matrice de réception apte à recevoir des cellules pancréatiques comprenant :
- une paroi semi-perméable délimitant au moins en partie un volume intérieur,
- un corps poreux, de préférence à base d'au moins un polymère, disposé dans le volume intérieur, comprenant :
   ∘ un premier jeu de cavité destinée à recevoir des cellules pancréatiques,
   ∘ un deuxième jeu de cavité non destinée à recevoir des cellules pancréatiques,
   ∘ le premier jeu de cavité et le deuxième jeu de cavité n'étant pas raccordés fluidiquement l'un à l'autre.

### BREVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
Les figures 1 et 2 représentent une vue en coupe transversale de la matrice selon deux exemples de réalisation.
Les figures 3 à 5 représentent une vue en coupe longitudinale d'un dispositif comprenant la matrice selon trois exemples de réalisation.
Les figures 6A à 6C représentent une vue de détail du dispositif comprenant le module fluidique illustré en figure 5, selon trois exemples de réalisation.
La figure 7 représente une vue en coupe transversale d'un dispositif comprenant la matrice selon un autre exemple de réalisation du module fluidique.
La figure 8 représente une vue en coupe transversale d'un dispositif comprenant la matrice et des électrodes pour l'électrolyse du liquide corporel, selon un exemple de réalisation.
Les figures 9A et 9B représentent deux vues d'un dispositif comprenant la matrice et des électrodes pour l'électrolyse du liquide corporel, selon un autre exemple de réalisation.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques. En particulier, les dimensions relatives ne sont pas représentatives de la réalité.

### DESCRIPTION DÉTAILLÉE DE L'INVENTION

Avant d'entamer une revue détaillée de modes de réalisation de l'invention, sont énoncées ci-après des caractéristiques optionnelles qui peuvent éventuellement être utilisées en association ou alternativement.

Selon un exemple, le premier jeu de cavité comprend au moins une cavité, et de préférence plusieurs cavités. Ces cavités peuvent ou non être interconnectées ou de façon équivalente raccordées fluidiquement entre elles.

Selon un exemple, le deuxième jeu de cavité comprend au moins une cavité, et de préférence plusieurs cavités. Ces cavités peuvent ou non être interconnectées ou de façon équivalente raccordées fluidiquement entre elles.

Selon un exemple, pour deux cavités consécutives du premier jeu prises selon une première direction, et de préférence pour chaque pour deux cavités consécutives du premier jeu prises selon la première direction, une cavité du deuxième jeu est disposée entre les deux cavités consécutives du premier jeu. Les cavités comprenant des cellules sont ainsi alternées selon la première direction avec des cavités exemptes de cellules, pour distribuer les zones de réserve dans la matrice. La première direction est de préférence perpendiculaire à la direction d'extension principale desdites cavités consécutives du premier jeu.

Selon un exemple, pour deux cavités consécutives du premier jeu prises selon une deuxième direction, et de préférence pour chaque deux cavités consécutives du premier jeu prises selon une deuxième direction, une cavité du deuxième jeu est disposée entre les deux cavités consécutives du premier jeu. Les cavités comprenant des cellules sont ainsi alternées avec des cavités exemptes de cellules selon deux directions. Les cavités comprenant des cellules peuvent ainsi être entourées de zones de réserves. La distribution des zones de réserve est donc encore améliorée, pour faciliter l'accès des cellules aux nutriments et aux gaz. La deuxième direction est de préférence perpendiculaire à la direction d'extension principale desdites cavités consécutives du premier jeu. De préférence, la première direction est sensiblement perpendiculaire à la deuxième direction.

Selon un exemple, une distance D1 entre au moins une cavité du deuxième jeu et au moins une cavité du premier jeu directement avoisinante, de préférence de chaque cavité du premier jeu directement avoisinante, prise du centre de la cavité du premier jeu au centre de l'au moins une cavité du deuxième jeu, est sensiblement supérieure ou égale à 0,5 mm (10⁻³ m), de préférence à 1,5 mm. La distance D1 peut être sensiblement inférieure ou égale à 3 cm (10⁻² m). La distance D1 peut être sensiblement comprise entre 0,5 mm et 3 cm.

Selon un exemple, la distance D1 entre chaque cavité du deuxième jeu et au moins une cavité du premier jeu directement avoisinante, de préférence de chaque cavité du premier jeu directement avoisinante, prise du centre de la cavité du premier jeu au centre de l'au moins une cavité du deuxième jeu, est sensiblement supérieure ou égale à 0,5 mm (10⁻³ m), de préférence à 1,5 mm. La distance D1 peut être sensiblement inférieure ou égale à 3 cm (10⁻² m). La distance D1 peut être sensiblement comprise entre 0,5 mm et 3 cm.

Selon un exemple, le premier jeu comprend une pluralité de cavités interconnectées et raccordées fluidiquement entre elles. Selon un exemple, le premier jeu comprend une pluralité de cavités interconnectées et raccordées fluidiquement, de préférence directement, à un canal d'injection. Les cavités du premier jeu sont par exemple interconnectées en étant raccordées fluidiquement, de préférence directement, au canal d'injection. La délivrance d'insuline améliorée sur le long terme en autorisant un renouvellement des cellules.

Selon un exemple, le deuxième jeu comprend une pluralité de cavités interconnectées. Selon un exemple, le deuxième jeu comprend une pluralité de cavités interconnectées et raccordées fluidiquement entre elles.

Selon un exemple, chaque cavité du premier jeu présente une section transversale de plus petite dimension, par exemple son diamètre, sensiblement supérieure à la taille des cellules pancréatiques ou des îlots de cellules pancréatiques. De façon synergique avec l'interconnexion des cavités du premier jeu, les cellules sont libres de circuler dans les cavités du premier jeu, ce qui facilite le renouvellement des cellules de la matrice.

Selon un exemple, les cavités du premier jeu s'étendent selon une direction d'extension principale, par exemple de façon parallèle entre elles.

Selon un exemple, les cavités du deuxième jeu s'étendent selon une direction d'extension principale, par exemple de façon parallèle entre elles.

Selon un exemple, les cavités du premier et du deuxième jeu s'étendent selon une direction d'extension principale, par exemple de façon parallèle entre elles.

Selon un exemple, les cavités du premier et du deuxième jeu s'étendent selon leur direction d'extension principale sur une longueur sensiblement supérieure ou égale à 70 %, de préférence sensiblement supérieure ou égale à 80 %, de la longueur du corps poreux prise selon la même direction.

Selon un exemple, le corps poreux est à base ou fait de chitosane, de polyacrylamide, de poly(p-phenyl-p-phthalamide), de nanofibres d'aramide, d'alcool polyvinylique.

Selon un exemple, le corps poreux présente un seuil de coupure compris entre 5,8 kDa et 8 kDa.

Selon un exemple, la paroi semi-perméable présente un seuil de coupure sensiblement compris entre 5,8 kDa et 8 kDa.

Selon un exemple, les cellules pancréatiques sont comprises dans des îlots de cellules pancréatiques. De préférence les îlots sont microencapsulés.

Selon un exemple, le dispositif comprend en outre un module fluidique configuré pour créer un flux de liquide autour et/ou à l'intérieur de la matrice.

Selon un exemple, le module fluidique comprend un revêtement de la matrice à base d'un polymère électroactif, le revêtement étant apte à déformer la matrice. Selon un exemple, le revêtement est additionnel à la paroi de la matrice. Selon un exemple le revêtement présente un seuil de coupure sensiblement supérieure ou égal à 5,8 kDa, par exemple sensiblement compris entre 5,8 kDa et 8 kDa.

Selon un exemple, le module fluidique comprend une pompe, la pompe étant configurée pour induire un flux de liquide autour et/ou à l'intérieur de la matrice.

Selon un exemple, le module fluidique est fluidiquement raccordé au deuxième jeu de cavité, de préférence le deuxième jeu comprenant une pluralité de cavités. Selon un exemple, les cavités du deuxième jeu sont interconnectées, par exemple par un canal.

Selon un exemple, le module fluidique comprend un réservoir et une pompe, la pompe étant configurée pour induire un flux de liquide depuis le réservoir jusque dans les cavités du deuxième jeu.

Selon un exemple, le réservoir comprend au moins l'un parmi un nutriment des cellules pancréatiques et un composé anti-inflammatoire.

Selon un exemple, le module fluidique comprend une pompe configurée pour induire un flux de liquide depuis un liquide extérieur au dispositif, par exemple un liquide corporel, jusque dans les cavités du deuxième jeu.

Selon un exemple, le module fluidique comprend un régulateur configuré pour réguler au moins un paramètre de la formation du flux par le module fluidique.

Selon un exemple, le dispositif comprend en outre au moins une anode et au moins une cathode, et une source d'énergie électrique. L'anode et la cathode peuvent être électriquement raccordées à la source d'énergie électrique, de sorte que, en présence de liquide corporel, un circuit électrique fermé est formé de façon à produire du dihydrogène à la cathode et du dioxygène à l'anode, par électrolyse du liquide corporel. Les cellules pancréatiques sont ainsi préservées par une production de dioxygène et de dihydrogène par électrolyse, pour améliorer la libération d'insuline dans le milieu intérieur.

Selon un exemple, le dispositif est configuré pour électrolyser le liquide corporel uniquement sous forme liquide. Le liquide corporel électrolysé ne comprend alors pas de fraction gazeuse.

Selon un exemple, au moins l'une parmi la cathode et l'anode est disposée dans le volume intérieur délimité au moins en partie par la paroi semi-perméable de la matrice. Le dispositif est ainsi simplifié par rapport aux solutions existantes prévoyant deux dispositifs ou compartiments distincts pour l'électrolyse et les cellules entre lesquels les gaz d'électrolyse sont acheminés.

Selon un exemple, au moins l'une parmi la cathode et l'anode est disposée dans le corps poreux de la matrice. Selon un exemple, la cathode et l'anode sont disposées dans le corps poreux de la matrice.

Selon un exemple, au moins l'une parmi la cathode et l'anode est disposée sur, de préférence directement sur, le corps poreux de la matrice.

Selon un exemple, la cathode et l'anode sont disposées en contact, de préférence directement en contact, avec le pourtour extérieur du corps de la matrice.

Dans la suite de la description, le terme « sur » ou « en contact » ne signifie pas nécessairement « directement sur » ou « directement en contact ». Ainsi, lorsque l'on indique qu'une pièce ou qu'un organe A1 est en appui « sur » une pièce ou un organe B1, cela ne signifie pas que les pièces ou organes A1 et B1 soient nécessairement en contact direct avec l'autre. Ces pièces ou organes A1 et B1 peuvent être soit en contact direct soit être en appui l'une sur l'autre par l'intermédiaire d'une ou plusieurs autres pièces.

Dans la description détaillée qui suit, il pourra être fait usage de termes tels que « longitudinal », « transversal », « supérieur », « inférieur », « intérieur », « extérieur ». Ces termes doivent être interprétés de façon relative en relation avec la position normale d'utilisation de la matrice de réception et/ou du dispositif de pancréas artificiel. Par exemple, la notion de « interne » correspond aux faces ou éléments tournés vers l'intérieur de la matrice et/ou du dispositif. La notion de « externe » correspond aux faces ou éléments tournés vers l'extérieur de la matrice et/ou du dispositif. Par exemple, les cavités s'étendant selon une direction d'extension principale, « longitudinal » s'entend parallèlement à cette direction, et « transversal » s'entend perpendiculairement à cette direction.

L'expression « A fluidiquement connecté à B » ou encore « A fluidiquement raccordé à B » est synonyme de « A est en connexion fluidique avec B » et ne signifie pas nécessairement qu'il n'existe pas d'organe entre A et B. Ainsi, ces expressions s'entendent d'une connexion fluidique entre deux éléments, cette connexion pouvant ou non être directe. Cela signifie qu'il est possible qu'entre un premier élément et un deuxième élément qui sont fluidiquement connectés, un parcours d'un fluide existe par un ou des conduits ou cavités ou canaux, éventuellement un organe supplémentaire, ce parcours étant distinct de la simple diffusion du fluide à travers le matériau du corps poreux, ce parcours pouvant ou non comprendre d'autres éléments.

A l'inverse, le terme « fluidiquement connecté directement » s'entend d'une connexion fluidique directe entre deux éléments. Cela signifie qu'entre un premier élément et un deuxième élément qui sont fluidiquement connectés directement aucun autre élément n'est présent, autre qu'un conduit/cavité/canal ou plusieurs conduits/cavités/canaux.

On entend par un paramètre « sensiblement égal/supérieur/inférieur à » une valeur donnée, que ce paramètre est égal/supérieur/inférieur à la valeur donnée, à plus ou moins 10 % près, voire à plus ou moins 5 % près, de cette valeur.

On entend par un élément « à base » d'un matériau, un élément comprenant ce matériau et éventuellement d'autres matériaux. « A base » d'un matériau s'entend comme le fait que ledit matériau est majoritaire relativement aux éventuels autres matériaux.

Par porosité d'un élément ou d'un matériau, on entend le volume non occupé par la matière le composant, relativement au volume apparent de l'élément ou du matériau. Cette proportion volumique peut être occupée par le milieu environnant de l'élément ou du matériau, du vide, du gaz ou un liquide, par exemple de l'eau. Dans le cadre de la présente invention, la porosité du matériau s'entend de façon distincte aux cavités du premier jeu et du deuxième jeu.

Par « seuil de coupure » d'une membrane, d'un corps ou d'un organe, on entend la masse molaire seuil ou la dimension seuil pour laquelle au moins 90 %, de préférence au moins 95 %, de préférence au moins 99 %, plus préférentiellement encore 100 %, des espèces de masse molaire ou de dimension supérieure ou égale à la masse molaire seuil ou la dimension seuil sont bloquées par la membrane, le corps ou l'organe.

La matrice 1 de réception de cellules pancréatiques et le dispositif 2 implantable de pancréas artificiel la comprenant sont maintenant décrits selon plusieurs exemples de réalisation.

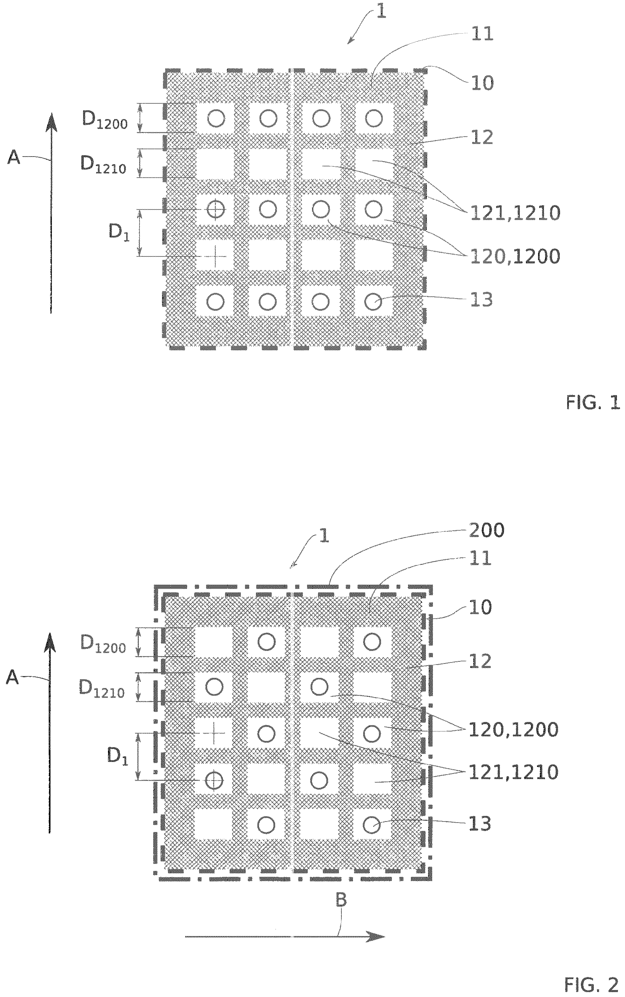

Le dispositif 2 de pancréas artificiel est destiné à être implanté dans le corps humain ou animal de façon à délivrer de l'insuline. Pour cela, le dispositif 2 comprend une matrice de réception 1 de cellules pancréatiques. La matrice 1 comprend, comme illustré par les figures 1 et 2, une paroi semi-perméable 10 délimitant au moins en partie un volume intérieur 11. Un corps poreux 12 est disposé dans le volume intérieur 11. Le corps poreux 12 est configuré pour accueillir des cellules pancréatiques 13, afin de délivrer de l'insuline. Les cellules pancréatiques 13 peuvent être isolées ou regroupées sous forme d'îlots pancréatiques. Un îlot pancréatique comprend au moins une cellule pancréatique, et de préférence une pluralité de cellules pancréatiques. Dans la suite, on considère à titre non limitatif que la matrice 1 réceptionne des îlots pancréatiques 13, aussi désignés îlots. Les cellules pancréatiques 13 sont par exemple des cellules bêta de Langerhans. Les cellules pancréatiques 13 peuvent être des cellules souches destinées à devenir des cellules pancréatiques ou bien des cellules pancréatiques issues de cellules souches.

Pour réceptionner les îlots 13, le corps poreux 12 comprend un premier jeu 120 de cavité 1200 et un deuxième jeu 121 de cavité 1210. Pour cela, le corps poreux 12 comprend des parois délimitant le premier jeu 120 de cavité 1200 et le deuxième jeu 121 de cavité 1210. Avantageusement, les parois sont formées par l'interface entre le corps poreux et les cavités. Préférentiellement, les parois ne sont pas formées par une couche de matériau supplémentaire. Par jeu de cavité, on entend un ensemble de cavité, l'ensemble comprenant au moins une cavité. Dans la suite, on considère à titre non limitatif que chaque jeu 120, 121 comprend une pluralité de cavités 1200, 1210. Les cavités 1200 du premier 120 comprennent les îlots 13, tandis que les cavités 1210 du deuxième jeu 121 ne comprennent pas d'îlot. Les cavités 1200 et les cavités 1210 ne sont pas raccordées fluidiquement entre elles, c'est à dire forment deux ensembles fluidiques distincts. De façon équivalente, les cavités 1200 ne communiquent pas fluidiquement avec les cavités 1210, sauf par la diffusion d'un liquide à travers les parois du corps poreux 12 et avantageusement au sein du corps poreux. Les cavités 1210 créent des chemins de diffusion des nutriments et des gaz dans la matrice 1, et forment ainsi des zones de réserve de nutriment et de gaz pour faciliter l'alimentation des îlots 13 compris dans les cavités 1200.

Comme par exemple illustré par les figures 1 et 2, dans la section transversale de la matrice 1, le corps poreux 12 peut former un réseau de cavités, par exemple sous la forme d'un maillage. Selon un exemple, les cavités 1200 et 1210 sont alternées de sorte que les cavités 1210 forment des zones de réserves entre les cavités 1200 comprenant les îlots 13. Comme par exemple illustré par la figure 1, selon une première direction A, une cavité 1210 peut être disposée entre deux cavités 1200 consécutives du premier jeu, de préférence entre chaque paire de cavité 1200 consécutives du premier jeu. Consécutives s'entend qui se suivent directement en ne considérant que les cavités 1200 du premier jeu 120. Les zones de réserves sont ainsi distribuées dans le corps poreux 12 selon la première direction A, entre les cavités 1200 accueillant les îlots 13. Le corps poreux 12, dans sa section transversale, peut comprendre au moins une rangée de cavités 1200 en alternance avec au moins une rangée de cavité 1210.

Comme par exemple illustré par la figure 2, une cavité 1210 peut être disposée entre deux cavités 1200 consécutives du premier jeu en outre selon une deuxième direction B, de préférence entre chaque paire de cavité 1200 consécutives du premier jeu. Consécutive s'entend là encore qui se suivent directement en ne considérant que les cavités 1200 du premier jeu 120. Les zones de réserves sont ainsi distribuées dans le corps poreux 12 selon la première direction A et la deuxième direction B, pour améliorer encore la répartition des zones de réserve et ainsi la diffusion des nutriments et des gaz aux îlots 13 compris dans les cavités 1200. Le corps poreux 12, dans sa section transversale, peut comprendre une alternance de cavités 1200 et de cavités 1210 selon ces deux directions. Selon la première direction A et/ou la deuxième direction B, et de préférence les deux, parmi les cavités qui l'entourent, une cavité 1200 du premier jeu 120 peut être entourée uniquement par des cavités 1210 du deuxième jeu 121 directement avoisinantes, et inversement pour une cavité 1210.

Selon un exemple, la première direction A et la deuxième direction B sont sensiblement perpendiculaires. La première direction A et la deuxième direction B peuvent être orthogonales à la direction d'extension principale des cavités 1200, 1210. La première direction A et la deuxième direction B peuvent être parallèles, et de préférence inclues, au plan d'une section transversale de la matrice 1.

La distance entre les cavités 1200, 1210 peut en outre être configurée de façon à permettre une bonne répartition des zones de réserves et des cavités 1200 accueillant les îlots 13 dans le corps poreux 12. Pour cela, la distance D1, par exemple selon la première direction A et/ou la deuxième direction B, entre une cavité 1210 et au moins une cavité 1200 directement avoisinante, et de préférence chaque cavité 1200 directement avoisinante, peut être sensiblement supérieure ou égale à 0,5 mm. Cette distance permet d'améliorer l'apport des nutriments pour les îlots 13 depuis la cavité 1210 dans la cavité 1200. Cette distance permet en outre une bonne tenue mécanique de la matrice. La distance D1 est prise du centre d'une cavité 1210 au centre de l'autre cavité 1210. Selon un exemple, les cavités 1200 et les cavités 1210 sont équidistantes entre elles. La distance D1 est par exemple prise dans le plan d'une section transversale de la matrice 1.

Selon un exemple, la distance D1 entre une cavité 1210 et au moins une cavité 1200 directement avoisinante, par exemple selon la première direction A et/ou la deuxième direction B, de préférence de chaque cavité 1200 directement avoisinante, est sensiblement inférieure ou égale à 3 cm, de préférence à 2 cm. Ainsi, l'encombrement associé à la matrice 1 peut être réduit pour un même nombre de cavités, diminuant ainsi une éventuelle gêne chez le patient implanté par le dispositif 2.

Les cavités 1200 du premier jeu 120 sont maintenant décrites. Selon un exemple, chaque cavité 1200 présente une section transversale présentant une plus petite dimension D1200 sensiblement supérieure ou égale à la taille des îlots 13. Selon un exemple, chaque cavité 1200 présente une section transversale présentant une plus petite dimension D1200 sensiblement supérieure ou égale à la taille d'au moins un îlot 13. De façon équivalente, la section transversale d'une cavité 1200 définit une forme dont la plus petite dimension D1200 est sensiblement supérieure ou égale à la taille des îlots 13. Ainsi, les cellules ne sont pas contraintes et fixées par les cavités 1200, ce qui facilite l'alimentation des îlots 13. La section transversale d'une cavité 1200 est plus particulièrement prise sensiblement perpendiculairement à sa direction d'extension principale.

Selon un exemple, les cavités 1200 présentant une section transversale circulaire, la dimension D1200 correspond au diamètre de la section transversale. Les cavités 1200 peuvent présenter une section transversale de toute autre forme, par exemple rectangulaire, carrée ou ellipsoïde.

À titre d'exemple, chez l'homme un îlot 13 présente une taille, par exemple un diamètre, de 130 µm en moyenne ; chez les petits animaux tels que la souris, un îlot 13 présente une taille, par exemple un diamètre, de 50 µm en moyenne. Selon un exemple, D1200 est sensiblement supérieure ou égale à 100 µm, de préférence 150 µm, de préférence 200 µm, de préférence 300 µm, et plus préférentiellement 400 µm. D1200 peut être sensiblement inférieure ou égale à 5 mm. Ainsi, l'encombrement associé à la matrice peut être réduit pour un même nombre de cavités, diminuant ainsi une éventuelle gêne chez le patient.

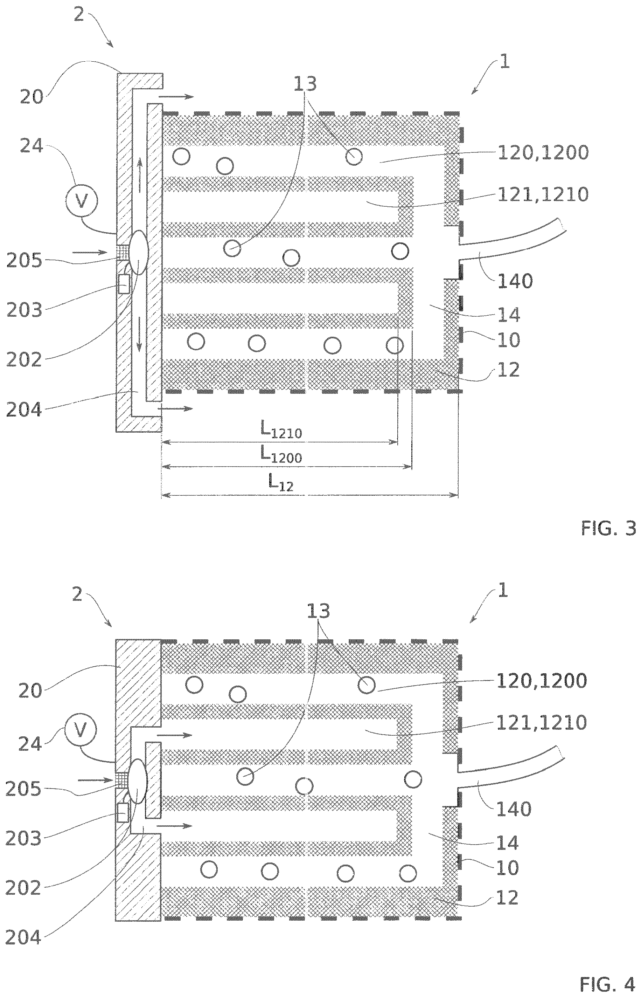

Les cavités 1200 du premier jeu 120 peuvent être fluidiquement non raccordées entre elles. De préférence, les cavités 1200 du premier jeu 120 sont interconnectées entre elles, c'est à dire raccordées fluidiquement entre elles. Les cavités 1200 du premier jeu 120 peuvent être interconnectées de façon à autoriser la circulation des îlots entre chaque cavité 1200 du premier jeu 120. De façon synergique avec la dimension D1200 des cavités, les îlots 13 sont ainsi libres de circuler entre les cavités 1200 du premier jeu 120. Une bonne répartition des îlots 13 peut ainsi être obtenue. Comme par exemple illustré en figure 3, les cavités 1200 peuvent être raccordées fluidiquement, de préférence directement, à un canal d'injection 14. Les îlots 13 peuvent ainsi être renouvelés par le biais d'une simple aspiration et d'une injection de nouveaux îlots dans le canal d'injection 14, sans nécessiter le retrait ou le remplacement de la matrice poreuse 1, et donc sans intervention chirurgicale. Le dispositif 2 comprenant la matrice 1 peut ainsi être implanté et délivrer de l'insuline à long terme. La délivrance d'insuline est par conséquent améliorée sur le long terme. Selon un exemple, le canal d'injection 14 est configuré de façon à raccorder fluidiquement, de préférence directement une extrémité de chaque cavité 1200. Selon un exemple, le canal d'injection 14 s'étend sensiblement perpendiculairement à la direction d'extension principale des cavités 1200.

Selon un exemple, le canal d'injection 14 est raccordée fluidiquement, de préférence directement, à une chambre d'injection 140. Les îlots peuvent ainsi être injectés depuis la chambre d'injection 140, jusque dans les cavités 1200. De préférence la chambre d'injection 140 est configurée pour présenter une extrémité de chargement disposée à l'extérieur du corps du patient, afin de permettre la recharge des îlots 13 par la chambre d'injection 140 depuis l'extérieur du corps du patient.

Comme par exemple illustré par la figure 3, les cavités 1200 peuvent s'étendre selon une direction principale de façon parallèle entre elles. Les cavités 1210 peuvent s'étendre selon une direction principale de façon parallèle entre elles. La structure du corps poreux est ainsi simplifiée, tout en étant compatible avec les alternances des cavités 1200 et 1210 décrites précédemment. Ces cavités peuvent s'étendre selon cette direction, sur une longueur L1200 et/ou L1210 sensiblement supérieure ou égale à 70 %, de préférence sensiblement supérieure ou égale à 80 %, de la longueur L12 du corps poreux 12 prise selon cette direction. Ainsi, une répartition des îlots 13 et/ou des zones de réserves est permise sur la longueur L12 du corps poreux 12.

Comme par exemple illustré par les figures 1 et 2, chaque cavité 1210 peut présenter une section transversale présentant une plus petite dimension D1210 sensiblement égale à la dimension D1200 des cavités 1200. Notons qu'on peut prévoir que D1210 soit inférieure ou supérieure à D1200. Selon un exemple, les cavités 1210 présentant une section transversale circulaire, la dimension D1210 correspond au diamètre de la section transversale. Les cavités 1210 peuvent présenter une section transversale de toute autre forme, par exemple rectangulaire, carrée ou ellipsoïde.

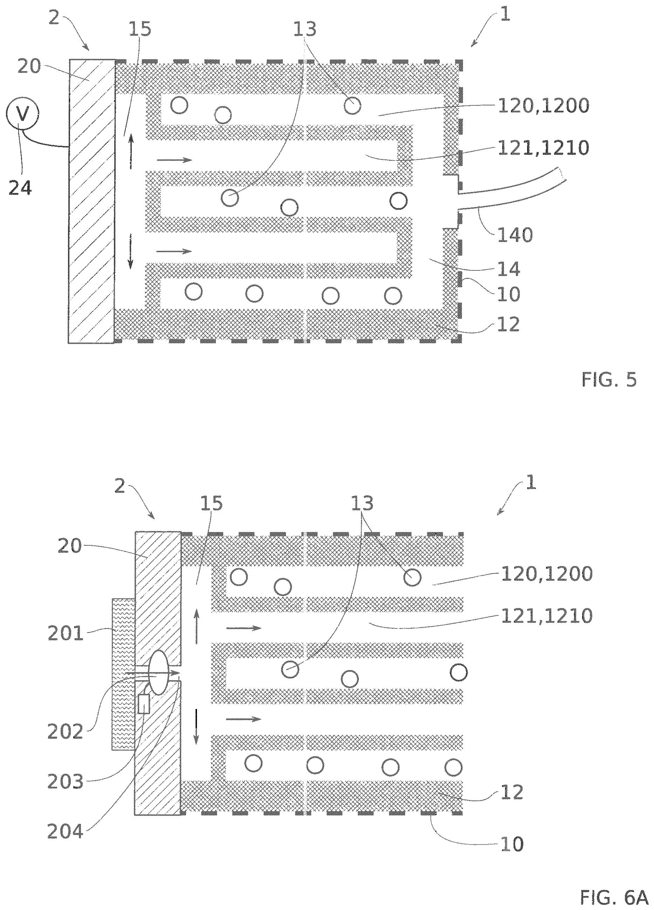

Les cavités 1210 du deuxième jeu 121 peuvent être fluidiquement non raccordées entre elles. De préférence, les cavités 1210 du deuxième jeu 121 sont interconnectées entre elles, c'est à dire raccordées fluidiquement entre elles. Les cavités 1210 créent ainsi des chemins de diffusion des nutriments et des gaz interconnectés favorisant une distribution homogène des nutriments et des gaz dans le corps poreux 12. Comme par exemple illustré en figure 5, les cavités 1210 peuvent être raccordées fluidiquement, de préférence directement, à un canal 15. Selon un exemple, le canal 15 est configuré de façon à raccorder fluidiquement, de préférence directement une extrémité de chaque cavité 1210. Selon un exemple, le canal 15 s'étend sensiblement perpendiculairement à la direction d'extension principale des cavités 1210. Le canal 15 et le canal d'injection 14 peuvent être situés l'un par rapport à l'autre à l'opposé du corps poreux 12 selon la direction principale d'extension des cavités 1200, 1210.

Le corps poreux 12 peut être à base ou fait d'un matériau apte à laisser passer les nutriments et les gaz, ainsi que l'insuline produite par les îlots, et à bloquer les îlots 13. Pour cela, le matériau peut présenter un seuil de coupure de préférence sensiblement supérieur ou égal à la masse moléculaire de l'insuline, soit environ 5,8 kDa (1 Da étant équivalent à 1 g/mol dans le système international des unités). Pour bloquer les îlots 13, le matériau du corps poreux 12 peut présenter un seuil de coupure de préférence sensiblement inférieur ou égal à la taille des îlots, par exemple leur diamètre moyen. Selon un exemple, le matériau du corps poreux peut présenter un seuil de coupure sensiblement inférieur ou égal à 100 µm, de préférence inférieur à 50 µm. De façon équivalente, le matériau du corps poreux 12 peut présenter une porosité apte à autoriser le passage des nutriments et les gaz, ainsi que l'insuline produite par les îlots, et à bloquer les îlots 13. La porosité peut être configurée pour laisser autoriser le passage des molécules de taille au moins égale à celle de l'insuline. Les nutriments et les gaz ayant une masse molaire ou moléculaire inférieure à 5,8 kDa, leur passage à travers le matériau du corps poreux 12 est autorisé. La porosité peut être configurée pour bloquer le passage des éléments de taille au moins égale à celle des îlots. Les gammes de valeurs présentées précédemment en lien avec le seuil de coupure du corps poreux 12 peuvent s'appliquer à la porosité du corps poreux 12.

Avantageusement, le corps poreux 12 présente un seuil de coupure configuré pour limiter voire empêcher le passage d'agents inflammatoires. Avantageusement, le corps poreux 12 présente un seuil de coupure configuré pour limiter voire empêcher la vascularisation dudit corps poreux.

Selon un exemple, le corps poreux 12 est à base d'un matériau apte à être imprimé en 3D. Selon un exemple, le corps poreux est à base d'un polymère naturel ou synthétique, de préférence biocompatible et non biodégradable. Le matériau peut être choisi parmi le chitosane, par exemple réticulé avec de la génépine pour devenir non biodégradable dans les conditions physiologiques, le polyacrylamide (PAAm), le poly(p-phenyl-p-phthalamide), des nanofibres d'aramide, l'alcool polyvinylique (PVA, de l'anglais polyvinylalcool).

La paroi semi-perméable 10 peut être à base ou faite d'un matériau apte à laisser passer les nutriments et les gaz, ainsi que l'insuline produite par les îlots, et à bloquer les molécules du système immunitaire, par exemple les cytokines. Pour cela, la paroi 10 peut présenter un seuil de coupure sensiblement compris entre 5,8 kDa et 8 kDa. Grâce un seuil de coupure compris entre 5,8 kDa et 8 kDa, la paroi externe permet une communication du liquide corporel entre le milieu intérieur du corps et le corps poreux 12 renfermant les îlots 13. Ainsi, les nutriments nécessaires aux îlots leur parviennent du milieu intérieur, et l'insuline produite par les îlots 13 peut être libérée dans ce milieu pour la régulation de la glycémie. Le passage à l'intérieur de la matrice 1 des molécules du système immunitaire, et notamment des cytokines, est bloqué par la paroi 11. Ainsi, les îlots sont protégés des réactions du système immunitaire. L'immunosuppression systémique chez le patient peut alors être limitée, et de préférence être évitée. Avantageusement, la paroi semi-perméable 10 est configurée pour réduire voire empêcher la vascularisation du corps poreux. La matrice 1 et/ou le dispositif 2 peut être configuré de sorte que la paroi semi-perméable 10 est directement au contact du corps du patient.

Selon un exemple, la paroi semi-perméable 10 est à base d'un polymère naturel ou synthétique, de préférence biocompatible et non biodégradable. De préférence la paroi semi-perméable 10 est à base d'un polymère présentant des propriétés d'anti-bioencrassement (communément désigné par le terme anglais *anti-biofouling*). Selon un exemple, la paroi est à base ou faite d'un hydrogel. Selon un exemple alternatif ou complémentaire, la paroi 10 est à base ou faite d'au moins un polymère parmi le polyéthylène glycol (PEG), l'alcool polyvinylique (PVA, de l'anglais polyvinylalcool), un copolymère (éthylène alcool vinylique) (EVOH, de l'anglais poly(ethylene vinyl alcohol)), le bromure d'hexadimethrine (en anglais hexadimethrine bromide, plus généralement connu sous le nom commercial de Polybrene) et la carboxymethyl cellulose. La paroi présente ainsi une bonne biocompatibilité et limite les phénomènes de bioencrassement.

Comme dit précédemment, la paroi semi-perméable 10 délimite au moins en partie le volume intérieur 11. En effet, la paroi semi-perméable 10 peut délimiter entièrement le volume intérieur 11, comme par exemple représenté par les figures 1 et 2. En alternative, la paroi semi-perméable 10 peut délimiter uniquement en partie le volume intérieur 11, comme par exemple représenté par les figures 3 à 5. Le volume intérieur 11 peut être délimité par la paroi semi-perméable 10 et un autre élément de la matrice 1 ou du dispositif 2, par exemple le module fluidique 20 décrit plus en détail ultérieurement. Le volume occupé par les îlots 13 peut être compris entre 2,5 mL et 5 mL (10⁻³ L). Le volume occupé par les îlots peut représenter sensiblement entre 25% et 50 % du volume intérieur 11. Le volume intérieur 11 peut avoir des dimensions représentant un volume total sensiblement égal ou inférieur à environ 20 mL, de préférence à, dans un ordre croissant de préférence 19 mL, 18 mL, 17 mL, 16 mL, 15 mL, 14, mL, 13 mL, 12 mL, 11 mL, 10 mL, 9 mL, 8 mL, 7 mL, 6 mL et 5 mL.

La matrice 1 est préférentiellement configurée en ce que le volume intérieur 11 soit exempt de vascularisation. La matrice 1 et plus particulièrement le corps poreux 12 peuvent être de toute forme, notamment parallélépipédique, cylindrique ou sous forme de disque. Le corps poreux 12 peut être monobloc, ou non. Le corps poreux 12 peut par exemple être formé par un assemblage de fibres poreuses et creuses, les parois des fibres délimitant les cavités 1200, 1210.

Selon un exemple, le nombre de cellules pancréatiques 13 contenues dans le corps poreux 12 est compris entre 10 000 et 50 000 000 cellules/kg par rapport au poids du patient. Ainsi, le nombre de cellules pancréatiques est adapté aux besoins en insuline du patient. Le nombre d'îlots 13 contenus dans le corps poreux 12 peut être sensiblement égal à 10 000 îlots/kg par rapport au poids du patient. Selon un exemple, considérant un poids moyen de 70 kg d'un patient type, le nombre de cellules pancréatiques 13 contenues dans le corps poreux 12 est compris entre 700 000 et 3 500 000 000 cellules/kg, soit environ 700 000 îlots pour un patient type de 70 kg.

Comme dit précédemment, les cellules pancréatiques 13 peuvent être comprises dans des îlots de cellules pancréatiques. De préférence les îlots sont microencapsulés. Ainsi une barrière supplémentaire de protection est disposée autour des cellules pour augmenter leur viabilité. Les îlots étant contenus dans la matrice, la récupération des îlots microencapsulés est facilitée par rapport aux solutions prévoyant une dissémination d'îlots microencapsulés dans le corps du patient.

Le dispositif 2 de pancréas artificiel est maintenant décrit en référence aux figures 2 à 9B. Le dispositif peut comprendre un module fluidique 20 configuré pour créer un flux de liquide autour et/ou à l'intérieur de la matrice 1. Le bioencrassement est ainsi limité par le flux de liquide en surface de la matrice et/ou de l'intérieur vers l'extérieur de la matrice. En outre, le flux de liquide à l'intérieur de la matrice 1 permet d'améliorer encore la diffusion des nutriments et des gaz dans le corps poreux 12.

Selon un exemple, le module fluidique 20 peut être configuré pour exercer une pression sur la matrice 1 de façon à la déformer ponctuellement, et par exemple à une certaine fréquence. Les contractions de la matrice 1 induisent ainsi des cisaillements fluidiques en surface de la matrice 1, ce qui limite le bioencrassement. Pour cela, le module fluidique peut comprendre comprend un revêtement 200 de la matrice 1. Le revêtement 200 peut recouvrir uniquement en partie la matrice 1. Le revêtement 200 peut recouvrir intégralement la matrice 1. Le revêtement 200 peut être additionnel à la paroi semi-perméable 10, par exemple en étant extérieur à la paroi 10 comme par exemple illustré par la figure 2, ou disposé entre la paroi 10 et le corps poreux 12. Selon un exemple alternatif, le revêtement 200 peut être formé par la paroi semi-perméable 10 de la matrice 1. Selon un exemple, le revêtement présente un seuil de coupure sensiblement supérieure ou égal à 5,8 kDa, par exemple sensiblement compris entre 5,8 kDa et 8 kDa, pour laisser passer l'insuline et les nutriments et bloquer les molécules du système immunitaire, par exemple les cytokines.

Le revêtement 200 peut être à base ou fait d'un polymère électroactif, connecté à une source d'énergie (non représentée en figure 2). Le polymère électroactif peut par exemple être choisi parmi : le copolymère (éthylène-vinyl acétate) (EVAc), le polyéthylène (PE), le polyaniline (PAni), par exemple le nigraniline (NA) et le leucoemeraldine (LM). Cette solution peut toutefois occasionner des forces de cisaillement pouvant impacter les organes environnants chez le patient. L'amplitude des contractions induites de la matrice 1 peut être choisie pour minimiser cet effet.

Selon un exemple alternatif ou complémentaire, le module fluidique 20 peut être configuré pour induire un flux de liquide autour de la matrice 1, et plus particulièrement en surface de la matrice 1, comme par exemple illustré par la figure 3. Le module fluidique 20 peut pour cela comprendre une pompe 202 configurée pour aspirer du liquide et injecter le liquide aspiré en surface de la matrice 1, par exemple par des conduits 204 débouchant en surface de la matrice 1.

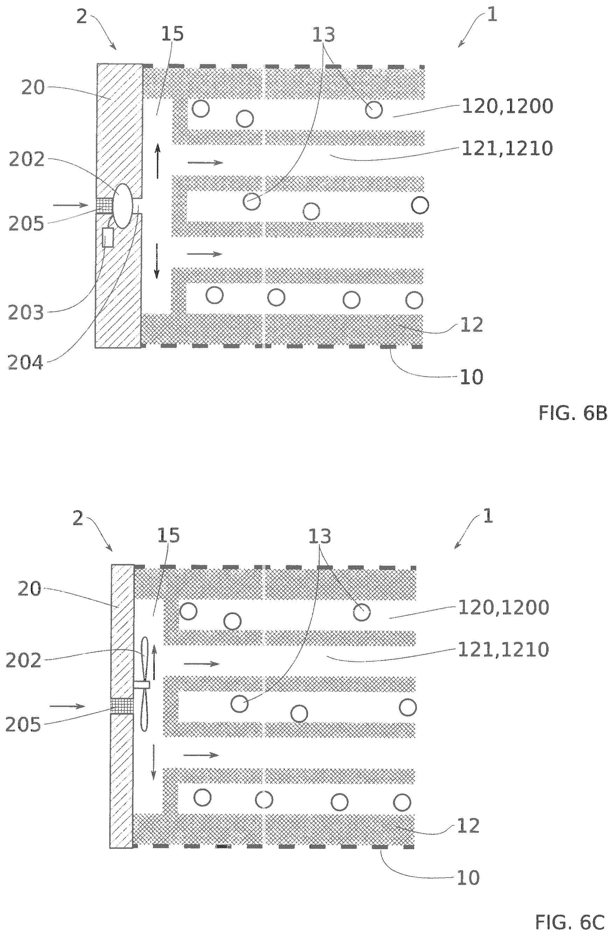

Selon un exemple alternatif ou complémentaire, le module fluidique 20 peut être fluidiquement raccordé au deuxième jeu 121 de cavité, le deuxième jeu 121 comprenant au moins une et de préférence plusieurs cavités 1210. Comme par exemple illustré par la figure 4, le module fluidique 20 peut être fluidiquement raccordé à la ou les cavités 1210, de préférence directement. Les cavités 1210 peuvent ne pas être interconnectées, comme l'illustre par exemple la figure 4. Les cavités 1210 peuvent être interconnectées. Comme par exemple illustré par les figures 5, 6A à 6C, le module fluidique 20 peut être raccordé fluidiquement, de préférence directement, à un canal 15 reliant les cavités 1210. Le module fluidique 20 peut être configuré pour induire un flux de liquide dans les cavités 1210. Le module fluidique 20 peut pour cela comprendre une pompe 202 configurée pour aspirer du liquide et injecter le liquide aspiré dans les cavités 1210, par exemple dans chacune des cavités 1210 séparément ou par l'intermédiaire du canal 15, par exemple par un ou plusieurs conduits 204 débouchant dans chaque cavité 1210, ou dans le canal 15. Comme par exemple illustré en figure 6C, la pompe 202 peut être disposée dans le canal 15.

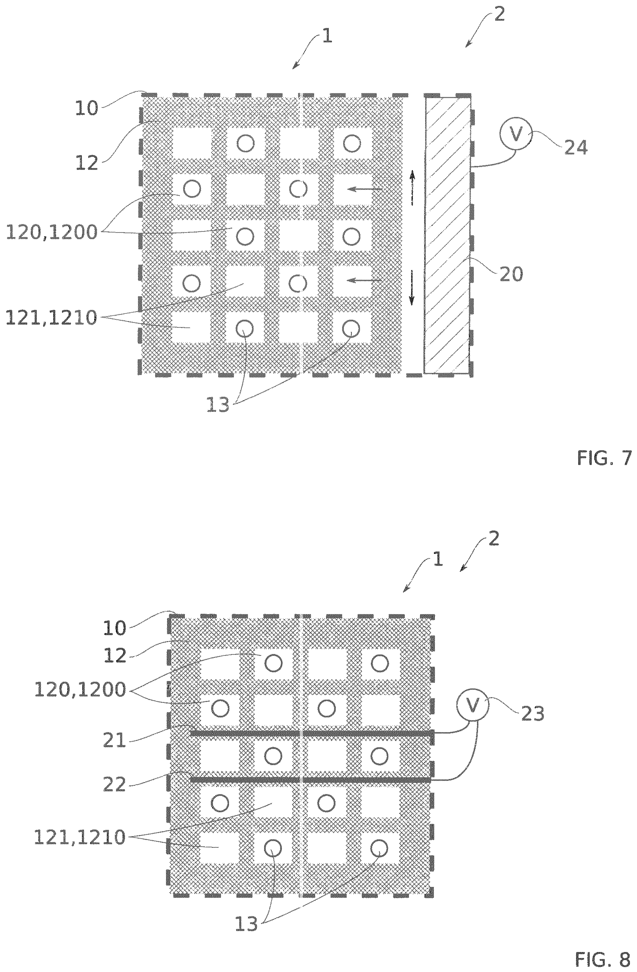

Selon un exemple alternatif ou complémentaire, le module fluidique 20 peut être configuré pour générer un flux transverse à la direction d'extension principale des cavités 1210, comme par exemple illustré par la figure 7. Le flux peut se propager à travers le matériau du corps poreux 12.

Le module fluidique 20 peut être configuré pour aspirer du liquide corporel extérieur au dispositif 2. De préférence, le module fluidique 20 comprend une membrane 205 configurée pour bloquer les molécules du liquide corporel pouvant encrasser le dispositif 2, par exemple le module fluidique 20 et plus particulièrement la pompe 202, comme par exemple illustré par les figure 3, 4, 6B et 6C. La membrane 205 peut pour cela présenter un seuil de coupure compris entre 20 et 300 Da. La membrane peut être à base ou faite de PVA, d'EVOH ou de chitosane.

Le module fluidique 20 peut être configuré pour aspirer du liquide issu d'un réservoir 201, et induire un flux de liquide depuis le réservoir 201 jusque dans les cavités 1210 et/ou en surface de la matrice 1. Notons que le module fluidique 20 illustré en figure 3 peut comprendre le réservoir 201, la pompe 202 pouvant être configurée pour induire un flux de liquide depuis le réservoir 201 jusqu'en surface de la matrice 1. Comme par exemple illustré par la figure 6A, la pompe 202 peut être configurée pour induire un flux de liquide depuis le réservoir 201 jusque dans les cavités 1210, qu'elles soient interconnectées ou non.

Selon un exemple, le réservoir 201 comprend au moins l'un parmi un nutriment des îlots 13 et un composé anti-inflammatoire. Le module fluidique 20 peut être configuré de sorte que les nutriments contenus dans le réservoir 201 sont injectés dans le corps poreux 12, et plus particulièrement dans les cavités 1210, pour fournir les zones de réserves et faciliter l'alimentation des îlots 13. Le ou les composés anti-inflammatoires permettent un traitement local anti-inflammatoire au niveau du dispositif 2. Le module fluidique 20 peut être configuré de sorte que le composé anti-inflammatoire contenu dans le réservoir 201 est injecté en surface de la matrice 1. Cela permet de limiter encore la quantité de composé anti-inflammatoire par rapport à un traitement systémique.

Selon un exemple, le réservoir 201 est configuré pour être rechargeable, par exemple par le biais d'une injection. Le réservoir 201peut par exemple être connecté à une chambre d'injection ou être rechargeable par le biais d'une injection pratiquée dans le réservoir. Le contenu du réservoir 201 peut ainsi être renouvelé.

Plusieurs types de pompes 202 peuvent être envisagés. À titre d'exemples non limitatifs, on peut prévoir que la pompe soit une pompe acoustique, ou bien une pompe mécanique, par exemple une hélice ou une valve mécanique. La pompe 202 peut être configurée pour générer un flux turbulent de liquide, afin par exemple d'augmenter les forces de cisaillement en surface, et/ou de mieux favoriser la diffusion des nutriments et des gaz pour alimenter les îlots 13. La pompe 202 peut être configurée pour générer un flux alternatif de liquide, c'est-à-dire pour injecter du liquide dans un premier sens selon une première configuration, et dans un sens opposé au premier sens selon une deuxième configuration. Ceci est particulièrement avantageux avec l'utilisation de la membrane 205, pour décoller les molécules pouvant s'accumuler sur la membrane 205 et gêner l'aspiration du liquide par la pompe 202.

Le module fluidique 20 peut en outre comprendre un régulateur 203 configuré pour réguler au moins un paramètre de la formation du flux par le module fluidique 20. Le régulateur 203 permet ainsi d'ajuster le flux formé par le module fluidique 20 selon les besoins, par exemple la vitesse du fluide et/ou les phases d'actionnement du module fluidique 20. Le régulateur 203 peut par exemple être configuré pour actionner le module fluidique 20, et notamment la pompe 202, de façon intermittente, entre des phases de génération de flux et des phases de non-génération de flux par le module fluidique 20. Le régulateur 203 peut par exemple être configuré pour moduler la vitesse du flux généré par le module fluidique 20, par exemple la vitesse de la pompe 202, de façon à varier l'intensité du flux de liquide formé. Ceci est particulièrement avantageux pour moduler le flux en fonction du temps suivant l'implantation du dispositif 2. Typiquement, le flux de liquide peut être plus intense et/ou généré plus fréquemment dans les premières semaines après l'implantation, puis il peut être ralenti ou sa fréquence peut être diminuée.

Le module fluidique 20, et plus particulièrement la pompe 202 et le cas échéant le régulateur 203, peuvent être alimentés électriquement par une source d'énergie électrique 24. Les sources d'énergie 24 pouvant être envisagées à titre d'exemple sont décrites ultérieurement.

Le dispositif 2 peut comprendre des électrodes : au moins une anode 21 et au moins une cathode 22, et une source d'énergie 23 électrique. L'anode 21 et la cathode 22 peuvent être électriquement raccordées à la source d'énergie 23 électrique, de sorte que, en présence de liquide corporel, un circuit électrique fermé est formé de façon à induire l'électrolyse de l'eau du liquide contenu dans le dispositif 2 ou dans son environnement, par exemple le liquide corporel. Du dihydrogène à la cathode 22 et du dioxygène à l'anode 21 peuvent être produits.

Une oxygénation des îlots est donc réalisée, couplée aux propriétés anti-inflammatoires de l'hydrogène permettant de diminuer les réactions immunitaires, et notamment les réactions inflammatoires post-transplantation. L'hydrogène est libéré dans le milieu intérieur en passant à travers la paroi semi-perméable de la matrice 1, pour limiter les réactions inflammatoires pouvant survenir autour du dispositif 2. Le dispositif 2 de pancréas artificiel permet de tirer parti des fonctions d'électrolyseur et de pancréas artificiel, tout en restant de structure simple.

De préférence, au moins l'une parmi la cathode 22 et l'anode 21 est disposée dans le volume intérieur 11, et de préférence les deux électrodes 21, 22 sont disposées dans le volume intérieur 11. L'électrolyse et la production d'insuline sont réalisées toutes deux dans le volume intérieur de la matrice, simplifiant ainsi le dispositif par rapport aux solutions prévoyant deux dispositifs ou compartiments distincts pour l'électrolyse et les cellules entre lesquels les gaz d'électrolyse sont acheminés. Le dispositif 2 est en outre plus compact et donc moins invasif pour le patient.

Selon un exemple, la cathode 22 et/ou l'anode 21 sont disposées dans le corps poreux 12 de la matrice 1, par exemple entre les cavités 1200, 1210. Plus particulièrement, la cathode 22 et/ou l'anode 21 peuvent être disposées dans le matériau du corps poreux 12. La cathode 22 et/ou l'anode 21 peuvent être disposées dans les parois du corps poreux 12 délimitant les cavités 1200, 1210, comme par exemple illustré par la figure 8. La production des gaz d'électrolyse est ainsi faite en proximité des cavités 1200 accueillant les îlots 13.

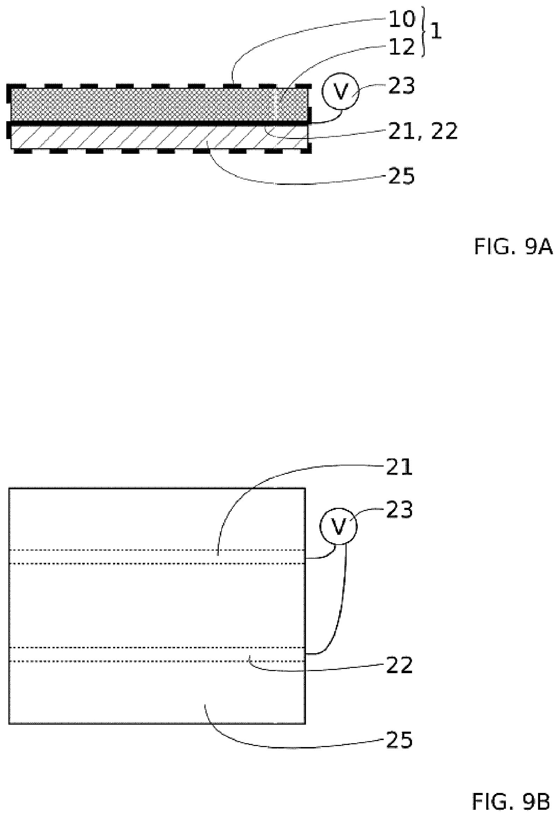

La cathode 22 et/ou l'anode 21 peuvent en alternative ou en complément être disposées sur, de préférence directement sur, le corps poreux 12 de la matrice 1. La cathode 22 et/ou l'anode 21 peuvent être disposées sur, de préférence directement sur, le pourtour extérieur du corps poreux 12, comme par exemple illustré par les figures 9A et 9B. La cathode 22 et/ou l'anode 21 peuvent être disposées sur un support solide 25. Selon l'agencement relatif des électrodes par rapport au corps poreux 12, on peut favoriser la délivrance aux îlots d'un certain gaz, notamment de l'oxygène, ou au milieu environnant, notamment pour l'hydrogène.

Les électrodes 21, 22 sont distantes l'une de l'autre de manière à permettre le fonctionnement du dispositif 2. La distance entre les électrodes est définie par la valeur du courant de l'électrolyse de l'eau. En effet, par exemple à la cathode, la réduction des protons crée une couche de déplétion dont l'épaisseur dépend de la valeur du courant de réduction. Dans tous les cas, la distance entre électrodes est de préférence supérieure à l'épaisseur de la couche de déplétion. Généralement, la distance les séparant peut être comprise entre environ 0,1 mm et environ 1 cm, notamment entre environ 0,2 et environ 7 mm, de préférence entre environ 0,5 et environ 5 mm. Cette distance de séparation peut s'appliquer entre deux électrodes massives (3D) disposées en parallèle, ou entre deux électrodes en deux dimensions (2D) supportées par deux supports parallèles ou il peut s'agir de la distance de séparation entre deux électrodes 2D disposées sur un même support.

Les électrodes peuvent avoir une géométrie en 2D. Ces électrodes en 2D peuvent être fabriquées par dépôt du matériau de l'électrode sur un support ou deux supports. On peut utiliser un seul support pour les deux électrodes à la condition que le support ne soit pas électriquement conducteur. Comme support, on peut citer une feuille fine de graphite, de platine ou d'or, une feuille fine permettant la diffusion des gaz dite « Gas diffusion Layer », une feuille de papier, de verre, de silicium. Le dépôt peut être: dépôt physique (par exemple PVD pour dépôt physique en phase vapeur, évaporation cathodique, lithographie, dépôt par plasma), électrochimique, impression, spray, ou compression mécanique, dépôt chimique ( par exemple CVD pour dépôt chimique en phase vapeur, sol-gel).

Les électrodes peuvent avoir une géométrie en 3D. Elles peuvent être formées classiquement, de préférence par compression, stéréolithographie, ou impression 3D.

La composition des électrodes est adaptée à la fonction de chacune d'elle. Elles peuvent être du même matériau ou de deux matériaux différents. Elles peuvent être à base ou faites de carbone. De préférence, le type de matériau de carbone est choisi parmi le graphite, les nanotubes de carbone, le graphène, du charbon actif ou du diamant. Le matériau des électrodes peut être dopé, notamment au platine, au fer ou à l'or. Les électrodes peuvent être à base ou faites de platine, d'or, d'oxyde d'indium et étain (communément abrégé ITO pour « indium tin oxide »), iridium ou en diamant dopé, en particulier au moins l'anode peut être en or, ou dopée à l'or. Les électrodes peuvent présenter une épaisseur sensiblement comprise entre 100 µm et 2 mm, selon une direction perpendiculaire à la face 101 destinée à être en contact avec la peau, notamment lorsque les électrodes sont sous la forme de plots, de barre ou de feuillet.

Les électrodes, notamment lorsqu'elles sont en métal, par exemple en or ou platine, peuvent aussi être des lames, par exemple de quelques centimètres de long, quelques millimètres de large, quelques dizaines ou centaines de microns d'épaisseur, ou des fils nus (catégorie des formes 3D).

Les électrodes peuvent en outre être chacune encapsulées par une membrane semi-perméable entourant chaque électrode, non représentée sur les figures, par exemple de type polyéther sulfone, polyamide, polyméthylméthacrylate (PMMA), chitosane, PVA. La membrane semi-perméable entourant l'anode 21 et/ou la cathode 22 peut être à base, et de préférence faite, de fluoropolymère à base de tétrafluoroéthylène sulfonaté, plus connu sous le nom de Nafion^{®}. Cette membrane semi-perméable peut présenter un seuil de coupure inférieur à 50 Da et entourer les électrodes 21, 22. La membrane est configurée pour empêcher le passage des composants du liquide corporel jusqu'aux électrodes, par exemple des nutriments essentiels aux îlots. Seuls les ions conducteurs, les molécules d'eau, ainsi que les gaz produits, peuvent circuler à travers cette membrane pour produire par électrode le dioxygène et le dihydrogène. Les réactions électrochimiques des composants du liquide corporel aux électrodes sont ainsi limitées, et de préférence empêchées. En outre, les phénomènes d'adsorption de ces composants à la surface des électrodes sont empêchés, évitant ainsi d'éventuelles réactions parasites. Les îlots 13 sont ainsi protégés de ces produits de réactions pouvant leur être néfastes.

Les sources d'énergie 23, 24 alimentant le dispositif 2 sont maintenant décrites. Ces sources peuvent être distinctes ou confondues. Dans la suite, on considère à titre non limitatif que les sources d'énergie 23, 24 sont confondues, c'est-à-dire qu'une source d'énergie alimente les différents éléments demandant une alimentation électrique. La source d'énergie 23, 24 peut être intégrée au dispositif. En alternative, la source d'énergie 23, 24 peut être déportée du dispositif 2 et reliée au dispositif 2 par des connexions électriques. La source d'énergie électrique représentant généralement un volume important de ce type de dispositif, en étant déportée elle peut être implantée à un endroit distinct du site d'implantation du dispositif 2, et ainsi minimiser la gêne occasionnée chez le patient. La source d'énergie peut être configurée pour être extérieure au corps du patient.

À titre non limitatif, la source d'énergie peut être :
- une pile, de préférence une pile à haute densité d'énergie, par exemple une pile au lithium,
- un dispositif de récupération d'énergie mécanique, exploitant par exemple l'effet piézoélectrique,
- une biopile capable de produire de l'électricité en consommant des espèces chimiques, typiquement naturellement présentes dans le corps humain ou animal, telles que : glucose, glucides, lipides, protéines,
- un dispositif de récupération d'énergie solaire, par exemple un module photovoltaïque ou une cellule Grätzel,
- un dispositif de récupération d'énergie thermique, par exemple un module thermoélectrique exploitant l'effet Seebeck.

La source d'énergie est de préférence capable de produire un voltage sensiblement inférieur ou égal à 1,4 V, afin d'éviter la formation de Cl₂ à partir des ions de Cl⁻.

Le dispositif 2 peut comprendre en outre un réducteur de tension qui permet d'obtenir un voltage d'alimentation de l'électrolyseur sensiblement inférieur ou égal à 1,4 V. Ceci est particulièrement utile lorsque la source d'énergie 23 reliée aux électrodes 21, 22 produit un voltage supérieur à 1,4 V. Le dispositif 2 peut comprendre plusieurs paires de cathodes 22 et d'anodes 21. La source d'énergie 23 peut être propre à chaque paire ou partagée.

Un procédé de fabrication de la matrice 1 est maintenant décrit selon un exemple. Le procédé peut comprendre la fabrication du corps poreux 12, par exemple par impression 3D. Le procédé peut comprendre l'ensemencement des cellules pancréatiques 13 dans le corps poreux 12. Après ou avant l'ensemencement, le procédé de fabrication peut comprendre l'encapsulation du corps poreux 12 par la paroi semi-perméable 10. Le procédé peut comprendre la fourniture d'une matrice 1 exempte de cellules pancréatiques, et comprendre l'ensemencement dans la matrice des cellules pancréatiques 13, par exemple par le biais du canal d'injection 14.

Un procédé de délivrance d'insuline est maintenant décrit. Le procédé peut comprendre l'implantation d'au moins une partie du dispositif 2 de pancréas artificiel, et plus particulièrement au moins de la matrice 1, dans un corps humain ou animal. Ceci peut notamment être fait par un acte chirurgical, adapté au site d'implantation et aux dimensions du dispositif. De préférence, l'implantation est réalisée au niveau d'un membre ou de l'abdomen.

Par animal au sens de l'invention, on peut notamment entendre les grands animaux tels que les bovins, les animaux de sport tel que le cheval, les animaux de compagnie comme les chiens et les chats, et les animaux de laboratoire tels que les rats, les souris et les singes.

Le procédé peut comprendre le raccordement électrique du module fluidique et/ou de l'au moins une anode et l'au moins une cathode à une source d'énergie électrique, notamment lorsque cette source est déportée. Le procédé peut aussi prévoir de commander la fermeture et l'ouverture du circuit électrique par des moyens prévus à cet effet.

Selon un exemple, le procédé comprend une recharge de cellules pancréatiques, par exemple par injection des cellules dans le canal d'injection de la matrice. Selon un exemple, le procédé comprend une aspiration des cellules pancréatiques, préalablement à la recharge.

Selon un exemple, le procédé comprend une recharge du réservoir du module fluidique, par exemple par injection.

Le procédé peut aussi prévoir de commander l'actionnement du module fluidique. Selon un exemple, le procédé comprend le réglage, par le régulateur, d'au moins un paramètre de la formation du flux par le module fluidique. Selon un exemple, le procédé comprend l'actionnement, et de préférence l'arrêt, du module fluidique du dispositif par le régulateur. Selon un exemple, le procédé comprend une modification par le régulateur de la vitesse du flux de liquide formé par le module fluidique du dispositif.

Au vu de la description qui précède, il apparaît clairement que l'invention propose une solution, et notamment une matrice de réception de cellules pancréatiques et un dispositif de pancréas artificiel permettant une délivrance d'insuline améliorée par rapport aux solutions existantes

L'invention n'est pas limitée aux modes de réalisations précédemment décrits et s'étend à tous les modes de réalisation couverts par l'invention. La présente invention ne se limite pas aux exemples précédemment décrits. Bien d'autres variantes de réalisation sont possibles, par exemple par combinaison de caractéristiques précédemment décrites, sans sortir du cadre de l'invention. En outre, les caractéristiques décrites relativement à un aspect de l'invention peuvent être combinées à un autre aspect de l'invention. Par exemple, un procédé peut comprendre une étape résultant de la mise en œuvre d'une caractéristique de la matrice et/ou du dispositif, et inversement.

### LISTE DES REFERENCES NUMERIQUES

1 Matrice de réception de cellules pancréatiques
10 Paroi semi-perméable
11 Volume intérieur
12 Corps poreux
120 Premier jeu de cavité
1200 Cavité
121 Deuxième jeu de cavité
1210 Cavité
13 Cellule pancréatique
14 Canal d'injection
140 Chambre d'injection
15 Canal fluidique
2 Dispositif de pancréas artificiel
20 Module fluidique
200 Revêtement électroactif
201 Réservoir
202 Pompe
203 Régulateur
204 Conduit
205 Membrane semi-perméable
21 Anode
22 Cathode
23 Source d'énergie électrique
24 Source d'énergie électrique
25 Support solide

## Revendications

1. Matrice (1) de réception de cellules pancréatiques comprenant :
• un premier jeu (120) de cavités (1200) comprenant des cellules pancréatiques (13),
• un deuxième jeu (121) de cavités (1210) exempt de cellule pancréatique,
• le premier jeu (120) de cavités et le deuxième jeu (121) de cavités n'étant pas raccordés fluidiquement l'un à l'autre autrement que par la diffusion d'un liquide à travers les parois d'un corps poreux (12) **caractérisée en ce qu'**elle comprend:
• une paroi (10) semi-perméable délimitant au moins en partie un volume intérieur (11),
• un corps poreux (12) disposé dans le volume intérieur (11), comprenant :
∘ le premier jeu (120), et
∘ le deuxième jeu (121).

2. Matrice (1) selon la revendication précédente, dans laquelle, pour chaque deux cavités (1200) consécutives du premier jeu (120) prises selon une première direction (A), une cavité (1210) du deuxième jeu (121) est disposée entre les deux cavités (1200) consécutives du premier jeu (120), et/ou pour chaque deux cavités (1200) consécutives du premier jeu (120) prises selon une deuxième direction (B), une cavité (1210) du deuxième jeu (121) est disposée entre les deux cavités (1200) consécutives du premier jeu (120).

3. Matrice (1) selon l'une quelconque des revendications précédentes, dans laquelle une distance (D1) entre au moins une cavité (1210) du deuxième jeu (121) et au moins une cavité (1200) du premier jeu (120) directement avoisinante prise du centre de la cavité (1200) du premier jeu (120) au centre de l'au moins une cavité (1210) du deuxième jeu (121), est comprise entre 0,5 mm et 3 cm.

4. Matrice (1) selon l'une quelconque des revendications précédentes, dans laquelle le premier jeu (120) comprend une pluralité de cavités (1200) interconnectées et raccordées fluidiquement à un canal d'injection (14) et/ou le deuxième jeu (121) comprend une pluralité de cavités (1210) interconnectées.

5. Matrice (1) selon l'une quelconque des revendications précédentes, dans laquelle chaque cavité (1200) du premier jeu (120) présente une section transversale de plus petite dimension (D₁₂₀₀) sensiblement supérieure à la taille des cellules pancréatiques (13) ou des îlots de cellules pancréatiques (13) et/ou les cavités (1200, 1210) du premier (120) et du deuxième jeu (121) s'étendent selon une direction d'extension principale de façon parallèle entre elles, sur une longueur (L1200, L1210) sensiblement supérieure ou égale à 70 %, de préférence sensiblement supérieure ou égale à 80 %, de la longueur (L12) du corps poreux (12).

6. Matrice (1) selon l'une quelconque des revendications précédentes, dans laquelle le corps poreux (12) est à base de chitosane, de polyacrylamide, de poly(p-phenyl-p-phthalamide), de nanofibres d'aramide, d'alcool polyvinylique.

7. Matrice (1) selon l'une quelconque des revendications précédentes, dans laquelle la paroi (10) présente un seuil de coupure sensiblement compris entre 5,8 kDa et 8 kDa.

8. Dispositif (2) de pancréas artificiel destiné à être implanté dans le corps humain ou animal et comprenant la matrice (1) de réception de cellules pancréatiques (13) selon l'une quelconque des revendications précédentes.

9. Dispositif (2) selon la revendication précédente, comprenant en outre un module fluidique (20) configuré pour créer un flux de liquide autour et/ou à l'intérieur de la matrice (1).

10. Dispositif (2) selon la revendication précédente, dans lequel le module fluidique (20) comprend un revêtement (200) de la matrice, par exemple formé par la paroi de la matrice, à base d'un polymère électroactif, le revêtement étant apte à déformer la matrice (1).

11. Dispositif (2) selon l'une quelconque des deux revendications précédentes, dans lequel le module fluidique (20) est fluidiquement raccordé au deuxième jeu (121) de cavité (1210), le deuxième jeu (121) comprenant de préférence une pluralité de cavités (1210) interconnectées, préférentiellement le module fluidique (20) comprend une pompe (202) configurée pour induire un flux de liquide depuis un liquide extérieur au dispositif jusque dans les cavités (1210) du deuxième jeu (121) et préférentiellement un régulateur (203) configuré pour réguler au moins un paramètre de la formation du flux par le module fluidique (20).

12. Dispositif (2) selon la revendication précédente, dans lequel le module fluidique (20) comprend un réservoir (201) et une pompe (202), la pompe (202) étant configurée pour induire un flux de liquide depuis le réservoir (201) jusque dans les cavités (1210) du deuxième jeu (121), préférentiellement le réservoir (201) comprend au moins l'un parmi un nutriment des cellules pancréatiques (13) et un composé anti-inflammatoire.

13. Dispositif (2) selon l'une quelconque des revendications 8 à 12, comprenant en outre au moins une anode (21) et au moins une cathode (22), une source d'énergie (23) électrique, l'anode (21) et la cathode (22) étant électriquement raccordées à la source d'énergie (23) électrique, de sorte que, en présence de liquide corporel, un circuit électrique fermé est formé de façon à produire du dihydrogène à la cathode (22) et du dioxygène à l'anode (21), par électrolyse du liquide corporel, au moins l'une parmi la cathode (22) et l'anode (21) étant disposée dans le volume intérieur (11) délimité au moins en partie par la paroi (10) de la matrice (1), préférentiellement la cathode (22) et l'anode (21) est disposée dans le corps poreux (12) de la matrice (1).

14. Dispositif (2) selon la revendication précédente, dans lequel au moins l'une parmi la cathode (22) et l'anode (21) est disposée sur, de préférence directement sur, le corps poreux (12) de la matrice (1).

15. Procédé d'ensemencement de la matrice (1) de réception de cellules pancréatiques comprenant :
a) une fourniture d'une matrice apte à former la matrice de réception (1) selon l'une quelconque des revendications 1 à 7, la matrice comprenant :
a1) une paroi (10) semi-perméable délimitant au moins en partie un volume intérieur (11),
a2) un corps poreux (12) disposé dans le volume intérieur (11), comprenant :
a21) un premier jeu (120) de cavités (1200),
a22) un deuxième jeu (121) de cavités (1210) exempt de cellule pancréatique,
a23) le premier jeu (120) de cavités et le deuxième jeu (121) de cavités n'étant pas raccordés fluidiquement l'un à l'autre autrement que par la diffusion d'un liquide à travers les parois d'un corps poreux (12)
b) un ensemencement du premier jeu de cavités de la matrice par des cellules pancréatiques.

## Patentansprüche

1. Aufnahmematrix (1) für Bauchspeicheldrüsenzellen, umfassend:
• einen ersten Satz (120) von Hohlräumen (1200), die Bauchspeicheldrüsenzellen (13) umfassen,
• einen zweiten Satz (121) von Hohlräumen (1210), die frei von Bauchspeicheldrüsenzellen sind,
• wobei der erste Satz (120) von Hohlräumen und der zweite Satz (121) von Hohlräumen nicht fluidisch miteinander verbunden sind, außer durch die Diffusion einer Flüssigkeit durch die Wände eines porösen Körpers (12) **dadurch gekennzeichnet, dass** sie umfasst:
• eine halbdurchlässige Wand (10), die mindestens teilweise ein Innenvolumen (11) begrenzt,
• einen porösen Körper (12), der im Innenvolumen (11) angeordnet ist und Folgendes umfasst:
∘ den ersten Satz (120), und
∘ den zweiten Satz (121).

2. Matrix (1) nach dem vorhergehenden Anspruch, wobei für jeweils zwei aufeinanderfolgende Hohlräume (1200) des ersten Satzes (120), die in einer ersten Richtung (A) aufgenommen werden, ein Hohlraum (1210) des zweiten Satzes (121) zwischen den beiden aufeinanderfolgenden Hohlräumen (1200) des ersten Satzes (120) angeordnet ist, und/oder für jeweils zwei aufeinanderfolgende Hohlräume (1200) des ersten Satzes (120), die in einer zweiten Richtung (B) aufgenommen werden, ein Hohlraum (1210) des zweiten Satzes (121) zwischen den beiden aufeinanderfolgenden Hohlräumen (1200) des ersten Satzes (120) angeordnet ist.

3. Matrix (1) nach einem der vorhergehenden Ansprüche, wobei ein Abstand (D1) zwischen mindestens einem Hohlraum (1210) des zweiten Satzes (121) und mindestens einem unmittelbar benachbarten Hohlraum (1200) des ersten Satzes (120) von der Mitte des Hohlraums (1200) des ersten Satzes (120) zur Mitte des mindestens einen Hohlraums (1210) des zweiten Satzes (121) zwischen 0,5 mm und 3 cm liegt.

4. Matrix (1) nach einem der vorhergehenden Ansprüche, wobei der erste Satz (120) eine Vielzahl von miteinander verbundenen und fluidisch mit einem Injektionskanal (14) verbundenen Hohlräumen (1200) umfasst und/oder der zweite Satz (121) eine Vielzahl von miteinander verbundenen Hohlräumen (1210) umfasst.

5. Matrix (1) nach einem der vorhergehenden Ansprüche, wobei jeder Hohlraum (1200) des ersten Satzes (120) einen Querschnitt mit einer kleineren Abmessung (D₁₂₀₀) aufweist, die im Wesentlichen größer ist als die Größe der Bauchspeicheldrüsenzellen (13) oder der Bauchspeicheldrüsenzelleninseln (13), und/oder die Hohlräume (1200, 1210) des ersten (120) und des zweiten Satzes (121) sich in einer Haupterstreckungsrichtung parallel zueinander über eine Länge (L1200, L1210) erstrecken, die im Wesentlichen größer oder gleich 70 %, vorzugsweise im Wesentlichen größer oder gleich 80 %, der Länge (L12) des porösen Körpers (12) ist.

6. Matrix (1) nach einem der vorhergehenden Ansprüche, wobei der poröse Körper (12) auf Chitosan, Polyacrylamid, Poly(p-phenyl-p-phthalamid), Aramid-Nanofasern, Polyvinylalkohol basiert.

7. Matrix (1) nach einem der vorhergehenden Ansprüche, wobei die Wand (10) eine Ausschlussgrenze aufweist, die im Wesentlichen zwischen 5,8 kDa und 8 kDa liegt.

8. Vorrichtung (2) für eine künstliche Bauchspeicheldrüse, die dazu bestimmt ist, in den menschlichen oder tierischen Körper implantiert zu werden, und die die Aufnahmematrix (1) für Bauchspeicheldrüsenzellen (13) nach einem der vorhergehenden Ansprüche umfasst.

9. Vorrichtung (2) nach dem vorhergehenden Anspruch, die ferner ein Fluidikmodul (20) umfasst, das so eingerichtet ist, dass es einen Flüssigkeitsstrom um und/oder innerhalb der Matrix (1) erzeugt.

10. Vorrichtung (2) nach dem vorhergehenden Anspruch, wobei das Fluidikmodul (20) eine Beschichtung (200) der Matrix umfasst, die beispielsweise durch die Wand der Matrix gebildet wird und auf einem elektroaktiven Polymer basiert, wobei die Beschichtung geeignet ist, die Matrix (1) zu verformen.

11. Vorrichtung (2) nach einem der beiden vorhergehenden Ansprüche, wobei das Fluidikmodul (20) fluidisch mit dem zweiten Satz (121) von Hohlräumen (1210) verbunden ist, wobei der zweite Satz (121) vorzugsweise eine Vielzahl von miteinander verbundenen Hohlräumen (1210) umfasst, wobei das Fluidikmodul (20) vorzugsweise eine Pumpe (202), die so eingerichtet ist, dass sie einen Flüssigkeitsstrom von einer außerhalb der Vorrichtung befindlichen Flüssigkeit in die Hohlräume (1210) des zweiten Satzes (121) induziert, und vorzugsweise einen Regler (203) umfasst, der so eingerichtet ist, dass er mindestens einen Parameter der Bildung des Stroms durch das Fluidikmodul (20) regelt.

12. Vorrichtung (2) nach dem vorhergehenden Anspruch, wobei das Fluidikmodul (20) ein Reservoir (201) und eine Pumpe (202) umfasst, wobei die Pumpe (202) so eingerichtet ist, dass sie einen Flüssigkeitsstrom von dem Reservoir (201) in die Hohlräume (1210) des zweiten Satzes (121) induziert, wobei das Reservoir (201) vorzugsweise einen Nährstoff für die Bauchspeicheldrüsenzellen (13) und/oder eine entzündungshemmende Verbindung umfasst.

13. Vorrichtung (2) nach einem der Ansprüche 8 bis 12, ferner umfassend mindestens eine Anode (21) und mindestens eine Kathode (22), eine elektrische Energiequelle (23), wobei die Anode (21) und die Kathode (22) elektrisch mit der elektrischen Energiequelle (23) verbunden sind, so dass bei Vorhandensein einer Körperflüssigkeit ein geschlossener Stromkreis gebildet wird, so dass durch Elektrolyse der Körperflüssigkeit an der Kathode (22) Diwasserstoff und an der Anode (21) Disauerstoff erzeugt werden, wobei die Kathode (22) und/oder die Anode (21) im Innenvolumen (11) angeordnet ist, das mindestens teilweise von der Wand (10) der Matrix (1) begrenzt ist, wobei die Kathode (22) und die Anode (21) vorzugsweise im porösen Körper (12) der Matrix (1) angeordnet sind.

14. Vorrichtung (2) nach dem vorhergehenden Anspruch, wobei die Kathode (22) und/oder die Anode (21) auf, vorzugsweise direkt auf, dem porösen Körper (12) der Matrix (1) angeordnet ist.

15. Verfahren zum Besiedeln der Aufnahmematrix (1) für Bauchspeicheldrüsenzellen, umfassend:
a) Bereitstellen einer Matrix, die geeignet ist, die Aufnahmematrix (1) nach einem der Ansprüche 1 bis 7 zu bilden, wobei die Matrix Folgendes umfasst:
a1) eine halbdurchlässige Wand (10), die mindestens teilweise ein Innenvolumen (11) begrenzt,
a2) einen porösen Körper (12), der im Innenvolumen (11) angeordnet ist und Folgendes umfasst:
a21) einen ersten Satz (120) von Hohlräumen (1200),
a22) einen zweiten Satz (121) von Hohlräumen (1210), die frei von Bauchspeicheldrüsenzellen sind,
a23) wobei der erste Satz (120) von Hohlräumen und der zweite Satz (121) von Hohlräumen nicht fluidisch miteinander verbunden sind, außer durch die Diffusion einer Flüssigkeit durch die Wände eines porösen Körpers (12)
b) Besiedeln des ersten Satzes von Hohlräumen der Matrix mit Bauchspeicheldrüsenzellen.

## Claims

1. Pancreatic-cell receiving matrix (1) comprising:
• a first set (120) of cavities (1200) comprising pancreatic cells (13),
• a second set (121) of cavities (1210) free from pancreatic cell,
• the first set (120) of cavities and the second set (121) of cavities being not fluidly connected to each other other than by diffusion of a liquid through the walls of a porous body (12)
**characterised in that** it comprises:
• a semi-permeable wall (10) delimiting at least partially an inner volume (11),
• a porous body (12) disposed in the inner volume (11), comprising:
∘ the first set (120), and
∘ the second set (121).

2. Matrix (1) according to the preceding claim, wherein, for each two consecutive cavities (1200) of the first set (120) taken in a first direction (A), a cavity (1210) of the second set (121) is disposed between the two consecutive cavities (1200) of the first set (120) and/or, for each two consecutive cavities (1200) of the first set (120) taken in a second direction (B), a cavity (1210) of the second set (121) is disposed between the two consecutive cavities (1200) of the first set (120).

3. Matrix (1) according to any one of the preceding claims, wherein a distance (D1) between at least one cavity (1210) of the second set (121) and at least one directly adjacent cavity (1200) of the first set (120) taken from the centre of the cavity (1200) of the first set (120) to the centre of the at least one cavity (1210) of the second set (121), is between 0.5 mm and 3 cm.

4. Matrix (1) according to any one of the preceding claims, wherein the first set (120) comprises a plurality of cavities (1200) interconnected and fluidically connected to an injection channel (14) and/or the second set (121) comprises a plurality of interconnected cavities (1210).

5. Matrix (1) according to any one of the preceding claims, wherein each cavity (1200) of the first set (120) has a cross-section with a smaller dimension (D₁₂₀₀) substantially larger than the size of the pancreatic cells (13) or pancreatic cell islands (13) and/or the cavities (1200, 1210) of the first (120) and second set (121) extend along a main extension direction parallel to each other, over a length (L1200, L1210) substantially greater than or equal to 70%, preferably substantially greater than or equal to 80%, of the length (L12) of the porous body (12).

6. Matrix (1) according to any one of the preceding claims, wherein the porous body (12) is based on chitosan, polyacrylamide, poly(p-phenyl-p-phthalamide), aramid nanofibers, or polyvinyl alcohol.

7. Matrix (1) according to any one of the preceding claims, wherein the wall (10) has a cut-off threshold substantially between 5.8 kDa and 8 kDa.

8. Artificial-pancreas device (2) intended to be implanted in the human or animal body and comprising the pancreatic-cell (13) receiving matrix (1)according to any one of the preceding claims.

9. Device (2) according to the preceding claim, further comprising a fluidic module (20) configured to create a flow of liquid around and/or within the matrix (1).

10. Device (2) according to the preceding claim, wherein the fluidic module (20) comprises a coating (200) of the matrix, for example formed by the wall of the matrix, based on an electroactive polymer, the coating being able to deform the matrix (1).

11. Device (2) according to any one of the two preceding claims, wherein the fluidic module (20) is fluidically connected to the second set (121) of cavity (1210), the second set (121) preferably comprising a plurality of interconnected cavities (1210), preferably the fluidic module (20) comprises a pump (202) configured to induce a flow of liquid from a liquid external to the device into the cavities (1210) of the second set (121) and preferentially a regulator (203) configured to regulate at least one parameter of flow formation by the fluidic module (20).

12. Device (2) according to the preceding claim, wherein the fluidic module (20) comprises a reservoir (201) and a pump (202), the pump (202) being configured to induce a flow of liquid from the reservoir (201) into the cavities (1210) of the second set (121), preferentially the reservoir (201) comprises at least one of a nutrient for the pancreatic cells (13) and an anti-inflammatory compound.

13. Device (2) according to any one of claims 8 to 12, further comprising at least one anode (21) and at least one cathode (22), and an electrical energy source (23), the anode (21) and the cathode (22) being electrically connected to the electrical energy source (23), so that, in the presence of body fluid, a closed electrical circuit is formed so as to produce dihydrogen at the cathode (22) and dioxygen at the anode (21), by electrolysis of the body fluid, at least one of the cathode (22) and the anode (21) being disposed in the inner volume (11) at least partially delimited by the wall (10) of the matrix (1), preferentially the cathode (22) and the anode (21) are disposed in the porous body (12) of the matrix (1).

14. Device (2) according to the preceding claim, wherein at least one of the cathode (22) and the anode (21) is disposed on, preferably directly on, the porous body (12) of the matrix (1).

15. Method for seeding the pancreatic-cell receiving matrix (1) comprising:
a) providing a matrix capable of forming the receiving matrix (1) according to any one of claims 1 to 7, the matrix comprising:
a1) a semi-permeable wall (10) delimiting at least partially an inner volume (11),
a2) a porous body (12) disposed in the inner volume (11), comprising:
a21) a first set (120) of cavities (1200),
a22) a second set (121) of cavities (1210) free from pancreatic cell,
a23) the first set (120) of cavities and the second set (121) of cavities being not fluidly connected to each other other than by diffusion of a liquid through the walls of a porous body (12)
b) seeding the first set of cavities of the matrix by pancreatic cells.
